# EUROPEAN PATENT SPECIFICATION

(11) **EP 3 265 127 B1**
(45) Date of publication and mention of the grant of the patent: **21.02.2024**
(21) Application number: 16759295.5
(22) Date of filing: 25.02.2016
(51) Int. Cl.: A61K 45/06, C07D 413/06, A61P 35/00, A61K 38/06, A61K 38/12, A61K 51/08, A61K 31/437, A61K 31/4745, A61K 31/505, A61K 31/513, A61K 31/567, A61P 3/10, A61K 38/08

(54) **MIFEPRISTONE AND OCTREOTIDE FOR USE IN THE TREATMENT OF CUSHING'S SYNDROME IN A PATIENT HAVING AN ADRENOCORTICOTROPIC HORMONE (ACTH)-SECRETING PANCREATIC NEUROENDOCRINE TUMOR**
MIFEPRISTON UND OCTREOTID ZUR VERWENDUNG BEI DER BEHANDLUNG DES CUSHING-SYNDROMS BEI EINEM PATIENTEN MIT EINEM ADRENOCORTICOTROPEN HORMON (ACTH) SEZERNIERENDEN PANKREATISCHEN NEUROENDOKRINEN TUMOR
MIFEPRISTONE ET OCTREOTIDE POUR LE TRAITEMENT DU SYNDROME DE CUSHING CHEZ UN PATIENT AYANT UNE TUMEUR NEUROENDOCRINE PANCRÉATIQUE SÉCRÉTANT L'HORMONE ADRÉNOCORTICOTROPE (ACTH)

(30) Priority: 02.03.2015 US 201562127153 P
(43) Date of publication of application: 10.01.2018
(73) Proprietor: Corcept Therapeutics, Inc., Menlo Park CA 94025 (US)
(72) Inventor: MORAITIS, Andreas G., Menlo Park, California 94025 (US); BELANOFF, Joseph K., Menlo Park, California 94025 (US)
(74) Representative: Geling, Andrea
(86) International application number: PCT/US2016/019646
(87) International publication number: WO 2016/140867

(56) References cited:
- WO-A1-2013/039916
- WO-A1-2013/039916
- WO-A2-2013/177559
- WO-A2-2013/177559
- US-A- 6 011 025
- US-A- 6 150 349
- US-A1- 2007 281 928
- US-A1- 2012 220 565
- US-A1- 2013 072 486
- US-A1- 2014 038 926
- DAVID E SCHTEINGART: "Drugs in the medical treatment of Cushing's syndrome - an update on mifepristone and pasireotide", EXPERT OPINION ON EMERGING DRUGS, vol. 17, no. 3, 25 April 2012 (2012-04-25) , pages 279-283, XP055489419, UK ISSN: 1472-8214, DOI: 10.1517/14728214.2012.679263
- HAZEL J. HUNT ET AL: "Discovery of a novel non-steroidal GR antagonist with in vivo efficacy in the olanzapine-induced weight gain model in the rat", BIOORGANIC & MEDICINAL CHEMISTRY LETTERS, vol. 22, no. 24, 1 December 2012 (2012-12-01), pages 7376-7380, XP055136322, ISSN: 0960-894X, DOI: 10.1016/j.bmcl.2012.10.074
- ANDREAS G. MORAITIS ET AL: "Mifepristone Improves Octreotide Efficacy in Resistant Ectopic Cushing's Syndrome", CASE REPORTS IN ENDOCRINOLOGY, vol. 2016, 1 January 2016 (2016-01-01), pages 1-5, XP055489443, ISSN: 2090-6501, DOI: 10.1155/2016/8453801

## Description

### BACKGROUND OF THE INVENTION

Adrenocorticotropic hormone (ACTH) is a polypeptide-based hormone that is normally produced and secreted by the anterior pituitary gland. ACTH stimulates secretion of cortisol and other glucocorticoids by specialized cells of the adrenal cortex. In healthy mammals, ACTH secretion is tightly regulated. ACTH secretion can be positively regulated by corticotropin releasing hormone (CRH), which is released by the hypothalamus. ACTH secretion can be negatively regulated by cortisol and other glucocorticoids.

Aberrant ACTH-levels can lead to a wide variety of undesirable physiological conditions. For example, excess ACTH levels can cause excess secretion of cortisol, resulting in hypercortisolemia or Cushing's Syndrome. Excess ACTH can result from aberrant secretion of ACTH from tumors or other dysregulated cells.

ACTH-secreting tumors arising from pituitary corticotroph cells (Cushing's disease) exhibit poor prognosis, and cause hypercortisolemia. Similarly, non-pituitary or ectopic ACTH-secreting tumors can also cause Cushing's Syndrome. ACTH-secreting tumors can increase ACTH levels in a subject, resulting in excess cortisol secretion that can be associated with osteoporosis, infections, psychiatric disorders, muscle atrophy, fat accumulation, hypertension, hyperglycemia, or ultimately death.

ACTH-secreting pituitary tumors are generally treated by transsphenoidal pituitary tumor resection, pituitary-directed radiation, adrenalectomy and/or medical suppression of adrenal gland cortisol production. While transsphenoidal ACTH-secreting tumor resection yields 30-70% surgical cure rate, adenoma recurrence rate is high. Efficacies of other therapeutic modalities are limited by factors such as slow therapeutic response, development of pituitary insufficiency, and uncontrolled pituitary tumor growth in the face of adrenal gland resection or inhibition. Effective pharmacotherapy directly targeting corticotroph tumor growth and/or ACTH secretion remains a major challenge. Therefore, novel therapies are urgently needed for treating ACTH-secreting tumors.

### BRIEF SUMMARY OF THE INVENTION

In one aspect, the present invention provides the glucocorticoid receptor antagonist (GRA) mifepristone and the somatostatin analog (SSA) octreotide for use in the treatment of Cushing's syndrome in a patient having an adrenocorticotropic hormone (ACTH)-secreting pancreatic neuroendocrine tumor, wherein the octreotide is administered first followed by a second administration of mifepristone. In some embodiments, the patient suffers from Cushing's Disease. In some embodiments, the patient suffers from ectopic ACTH Syndrome. In some embodiments, the method comprises administering the GRA mifepristone and the SSA octreotide for at least two weeks (*e*.*g*., from two weeks to 1, 2, 3, 4, 5, 6, 7, 8, 9, 10, 11, 12, or more months).

In some cases, the somatostatin analog octreotide is radiolabeled. In some cases, the radiolabeled somatostatin analog octreotide is radiolabeled with a label suitable for imaging, such as, *e*.*g*., ¹¹¹In or ¹²³I. In some cases, the somatostatin analog octreotide is radiolabeled with a label suitable for radionuclide therapy, such as, *e*.*g*., ¹¹¹In, ¹³¹I, ⁹⁰Y, ¹⁷⁷Lu, or ²¹³Bi. In some cases, the therapeutic radionuclide is selected from the group consisting of ¹¹¹In, ⁹⁰Y, ¹⁷⁷Lu, and ²¹³Bi. In some cases, the therapeutic radionuclide is selected from the group consisting of ⁹⁰Y, ¹⁷⁷Lu, and ²¹³Bi. In some cases, the somatostatin analog octreotide is labeled with a radionuclide selected from the group consisting of ³²P, ⁴⁵Ti, ⁴⁸V, ⁴⁹V, ⁵⁹Fe, ⁶⁰Cu, ⁶¹Cu, ⁶²Cu, ⁶⁴Cu, ⁶⁵Zn, ⁶⁷Cu, ⁶⁷Ga, ⁶⁸Ga, ⁷¹As, ⁷²As, ⁷⁶As, ⁷⁶Br, ⁷⁷As, ⁸⁹Sr, ⁹⁰Y, ^{99m}Tc, ¹¹¹In, ^{117m}Sn, ¹²³I, ¹²⁵I, ¹³¹I, ¹⁴⁹Pm, ¹⁵³Gd, ¹⁵³Pm, ¹⁵³Sm, ¹⁶⁶Ho, ¹⁷⁷Lu, ¹⁸⁶Re, ¹⁸⁸Re, ²⁰¹Tl, ²⁰³Pb, ²⁰⁹Pb, ²⁰⁹Bi, ²¹¹At, ²¹²Bi, ²¹²Pb, ²¹³Bi, ²²³Ra, and ²²⁵Ac. In some cases, the somatostatin analog is ¹²³I-Tyr³-octreotide, ¹¹¹In-DTPA-*D*-Phe¹-octreotide, [¹¹¹In-DTPA⁰]octreotide, or [⁹⁰Y-DOTA, Tyr³]octreotide. In some cases, the subject in need thereof suffers from inoperable or metastatic ACTH-secreting pancreatic neuroendocrine tumor, or an inoperable and metastatic ACTH-secreting pancreatic neuroendocrine tumor.

In some cases, the somatostatin analog octreotide is administered in a sustained release formulation. In some cases, the somatostatin analog is administered as octreotide LAR.

### BRIEF DESCRIPTION OF THE DRAWINGS

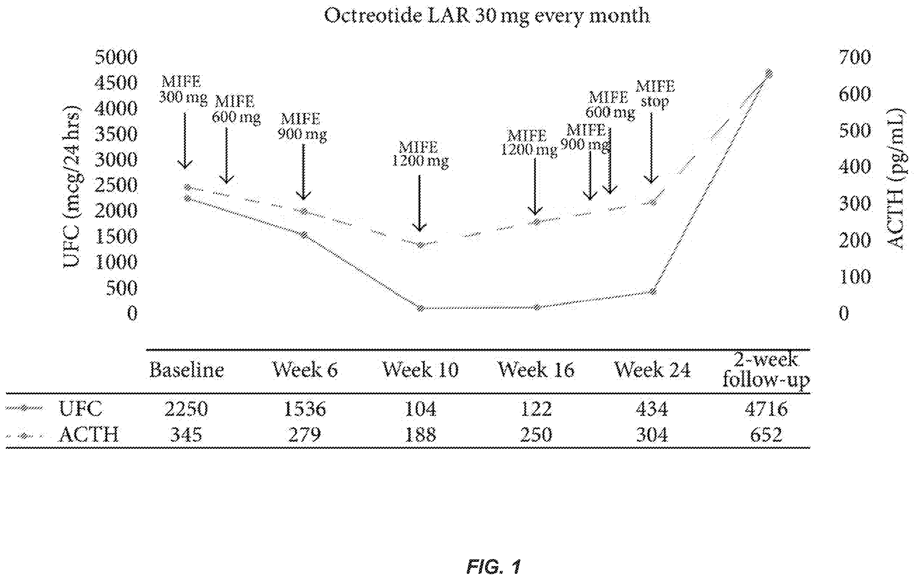

**Figure 1****:** illustrates the effect on adrenocorticotropic hormone (ACTH) and urinary free cortisol (UFC) levels of simultaneously or sequentially administering to a subject effective amounts of the glucocorticoid antagonist mifepristone (MIFE) and the somatostatin analog octreotide long-acting release (LAR). To convert values for UFC to nanomoles per 24 h, multiply by 2.76. To convert values for ACTH to picomoles per liter, multiply by 0.22.

### DETAILED DESCRIPTION OF THE INVENTION

### I. Introduction

In the context of the present invention, any statement about a method of treatment is to be construed as referring to the indicated compound(s) or composition(s) for use in the indicated method of treatment. The invention provides a novel treatment method for alleviating the effects of ACTH-secreting tumors by administering effective amounts of the glucocorticoid receptor antagonist (GRA) mifepristone and the somatostatin analog (SSA) octreotide.

### II. Definitions

The abbreviations used herein have their conventional meaning within the chemical and biological arts.

"Treat", "treating" and "treatment" refer to any indicia of success in the treatment or amelioration of a pathology or condition, including any objective or subjective parameter such as abatement; remission; diminishing of symptoms or making the pathology or condition more tolerable to the patient; slowing in the rate of degeneration or decline; making the final point of degeneration less debilitating; or improving a patient's physical or mental well-being. The treatment or amelioration of symptoms can be based on objective or subjective parameters; including the results of a physical examination; histopathological examination (*e*.*g*., analysis of biopsied tissue); laboratory analysis of urine, saliva, an inferior petrosal sinus sample, serum, plasma, or blood (*e*.*g*., to detect cortisol or ACTH levels); or imaging (*e*.*g*., imaging of ACTH-secreting tumor or detectably labeled somatostatin analog). Effective treatment refers to a reduction in ACTH-secretion, a reduction in ACTH or cortisol levels, a reduction in ACTH-secreting tumor burden (*e*.*g*., ACTH-secreting tumor size, mass, volume, viability, or proliferation), or an increase in ACTH-secreting tumor cell death.

"Patient" or "subject in need thereof' refers to a person having, or suspected of having an ACTH-secreting neuroendocrine tumor. An ACTH-secreting tumor can be identified and/or monitored by detection of the tumor, or detection of symptoms caused by an ACTH-secreting tumor. A neuroendocrine tumor can be detected and/or monitored by detection of the tumor or detection of symptoms caused by the tumor.

As used herein, the term "ACTH-secreting tumor" refers to an adenoma, adenocarcinoma, neuroendocrine, pituitary, or other tumor that secretes ACTH. In some cases, the ACTH-secreting tumor can cause an increase in blood, plasma, or serum levels of ACTH or blood, plasma, serum, or urinary (*e*.*g*., urinary free) cortisol levels in a subject having the ACTH-secreting tumor as compared to a subject that does not have an ACTH-secreting tumor. In some cases, the ACTH-secreting tumor does not respond to suppression or negative regulation of ACTH secretion by cortisol or other glucocorticoid receptor agonists (*e*.*g*., dexamethasone).

As used herein, the term "simultaneously or sequentially administering" refers to administration of a GRA compound and somatostatin receptor ligand compound (*e*.*g*., somatostatin or somatostatin analog (SSA)) such that the two compounds are in the body at the same time in amounts effective to treat an ACTH-secreting tumor.

As used herein, the term "effective amount," "amounts effective," or "therapeutically effective amount" refers to an amount or amounts of one or more pharmacological agents effective to treat, eliminate, or mitigate at least one symptom of the disease being treated. In some cases, "effective amount," "amounts effective," or "therapeutically effective amount" can refer to an amount of a functional agent or of a pharmaceutical composition useful for exhibiting a detectable therapeutic or inhibitory effect. The effect can be detected by any assay method known in the art. In some cases, the amounts effective, or the like, refer to amounts effective to reduce ACTH levels. In some cases, the amounts effective, or the like, refer to amounts effective to reduce cortisol (*e*.*g*., serum cortisol, salivary cortisol, or urinary free cortisol) levels. In some cases, the amounts effective, or the like, refer to amounts effective to reduce ACTH levels or cortisol levels, or a combination thereof, by at least 10%, 20%, 30%, 40%, 50%, 60%, 75%, 90%, 99%, or more.

As used herein, the term "effective to reduce secretion of ACTH by the tumor" refers to a method, treatment, composition, or amount that can reduce secretion of ACTH by pituitary, neuroendocrine, or other tumor as compared to the secretion of ACTH by such a tumor in the absence of the method, treatment, composition, or amount.

"Pharmaceutically acceptable excipient" and "pharmaceutically acceptable carrier" refer to a substance that aids the administration of an active agent to and absorption by a subject and can be included in the compositions of the present invention without causing a significant adverse toxicological effect on the patient. Non limiting examples of pharmaceutically acceptable excipients include water, NaCl, normal saline solutions, lactated Ringer's, normal sucrose, normal glucose, binders, fillers, disintegrants, lubricants, coatings, sweeteners, flavors and colors, and the like. One of skill in the art will recognize that other pharmaceutical excipients are useful in the present invention.

"Glucocorticoid receptor" ("GR") refers to the type II GR which specifically binds to cortisol and/or cortisol analogs such as dexamethasone (*See, e.g.,* Turner & Muller, J Mol Endocrinol October 1, 2005 35 283-292). The GR is also referred to as the cortisol receptor. The term includes isoforms of GR, recombinant GR and mutated GR. Inhibition constants (Kᵢ) against the human GR receptor type II (Genbank: P04150) are between 0.0001 nM to 1,000 nM; preferably between 0.0005 nM to 10 nM, and most preferably between 0.001 nM to 1nM.

"Glucocorticoid receptor antagonist" refers to any composition or compound which partially or completely inhibits (antagonizes) the binding of a glucocorticoid receptor (GR) agonist, such as cortisol, or cortisol analogs, synthetic or natural, to a GR. A "specific glucocorticoid receptor antagonist" refers to any composition or compound which inhibits any biological response associated with the binding of a GR to an agonist. By "specific," the drug preferentially binds to the GR rather than other nuclear receptors, such as mineralocorticoid receptor (MR), androgen receptor (AR), or progesterone receptor (PR). It is preferred that the specific glucocorticoid receptor antagonist bind GR with an affinity that is 10x greater (1/10^{th} the K_{d} value) than its affinity to the MR, AR, or PR, both the MR and PR, both the MR and AR, both the AR and PR, or to the MR, AR, and PR. In a more preferred embodiment, the specific glucocorticoid receptor antagonist binds GR with an affinity that is 100x greater (1/100^{th} the K_{d} value) than its affinity to the MR, AR, or PR, both the MR and PR, both the MR and AR, both the AR and PR, or to the MR, AR, and PR.

As used herein, the phrase "steroidal backbone" in the context of glucocorticoid receptor antagonists containing such refers to glucocorticoid receptor antagonists that contain modifications of the basic structure of cortisol, an endogenous steroidal glucocorticoid receptor ligand. The basic structure of a steroidal backbone is provided as Formula I: 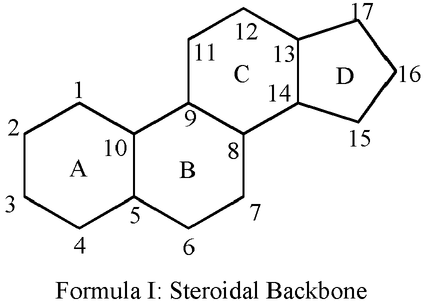 The two most commonly known classes of structural modifications of the cortisol steroid backbone to create glucocorticoid antagonists include modifications of the 11- β hydroxy group and modification of the 17- β side chain (*See, e. g.,* Lefebvre (1989) J. Steroid Biochem. 33: 557-563).

As used herein, the phrase "non-steroidal backbone" in the context of glucocorticoid receptor antagonists containing such refers to glucocorticoid receptor antagonists that do not share structural homology to, or are not modifications of, cortisol. Such compounds are described herein but not claimed and include synthetic mimetics and analogs of proteins, including partially peptidic, pseudopeptidic and non-peptidic molecular entities.

As used herein, the term somatostatin receptor refers to a class of G -protein coupled seven transmembrane receptors that bind somatostatin. There are five somatostatin receptor sub-types, referred to as SSTR1-SSTR5 respectively. See, *e.g.,* Trends Pharmacol Sci. 1995 Mar;16(3):86-8.

As used herein, the terms "somatostatin receptor ligand," or "somatostatin or somatostatin analog" refer to any ligand of any one of the somatostatin receptor subtypes (SSTR1-SSTR5). In some cases, which are described herein but not claimed, the ligand is somatostatin. Somatostatin is an inhibitory polypeptide with two primary biologically active forms SST14 and SST28. In some cases, which are described herein but not claimed, the ligand is a pre- or pre-pro form of somatostatin, or an analog thereof. In the present invention, the somatostatin ligand is the somatostatin analog octreotide. Somatostatin analogs can be agonists or antagonists of one or more somatostatin receptors. In some cases, the somatostatin ligand preferentially binds or activates somatostatin receptor type 2 (SSTR2). In some cases, the somatostatin receptor ligand preferentially binds or activates somatostatin receptor type 5 (SSTR5). In some cases, the somatostatin receptor ligand preferentially binds or activates SSTR2 and SSTR5. In some cases, the somatostatin receptor ligand preferentially binds or activates SSTR2, SSTR3, and SSTR5. The somatostatin receptor ligand octreotide can be provided or administered in a long acting, prolonged, or slow release formulation.

"Salt" refers to acid or base salts of the compounds used in the methods of the present invention. Illustrative examples of pharmaceutically acceptable salts are mineral acid (hydrochloric acid, hydrobromic acid, phosphoric acid, and the like) salts, organic acid (acetic acid, propionic acid, glutamic acid, citric acid and the like) salts, quaternary ammonium (methyl iodide, ethyl iodide, and the like) salts. It is understood that the pharmaceutically acceptable salts are non-toxic. Additional information on suitable pharmaceutically acceptable salts can be found in Remington's Pharmaceutical Sciences, 17th ed., Mack Publishing Company, Easton, Pa., 1985.

As used herein, the terms "sustained release," "slow release," "long acting," "prolonged release," and the like refer to a formulation containing at least one active ingredient (*e*.*g*., the somatostatin analog octreotide, the GRA mifepristone, or a combination thereof) formulated to maintain a therapeutic concentration of active ingredient(s) in a patient for a longer period of time in comparison to formulations that are not designed for such sustained release. In some cases, the sustained release formulation maintains therapeutic concentration of one or more active ingredient(s) for, or for at least, one week, two weeks, three weeks, four weeks, five weeks, or six weeks. In some cases, the sustained release formulation is administered to a patient every one, two, three, four, five, or six weeks. Exemplary sustained release formulations include, but are not limited to octreotide LAR.

### III. Methods

The present invention provides a method of treating Cushing's syndrome in a patient having an adrenocorticotropic hormone (ACTH)-secreting pancreatic neuroendocrine tumor in a subject in need thereof. The method comprises administering to the subject the glucocorticoid receptor antagonist GRA mifepristone and the somatostatin analog (SSA) octreotide, in amounts effective to reduce secretion of ACTH by the tumor. The SSA octreotide is administered first and then the GRA mifepristone is administered.

### A. ACTH-secreting tumors

### 1) Types of ACTH-secreting tumors

Methods and compositions described herein are useful for treating a wide variety of ACTH-secreting tumors. ACTH-secreting tumors include, but are not limited to, tumors that secrete ACTH and express one or more somatostatin receptors (*e*.*g.*, one of SSTR1-5, or a combination thereof). In some cases, expression of one or more somatostatin receptor subtypes (*e*.*g*., expression of one of SSTR1-5, or a combination thereof) is upregulated in an ACTH-secreting tumor after administration of a glucocorticoid receptor antagonist. In some cases, somatostatin receptor expression (*e*.*g*., expression of one of SSTR1-5, or a combination thereof) is undetectable prior to administration of a glucocorticoid receptor antagonist and detectable after administration of a glucocorticoid receptor antagonist. In some cases, the types of ACTH-secreting tumors that can be treated by the methods and compositions described herein include, but are not limited to, adenomas, adenocarcinomas, pituitary adenomas or pituitary adenocarcinomas, carcinoid tumors, neuroendocrine tumors, or combinations thereof.

The ACTH-secreting tumor can be a benign or malignant neuroendocrine tumor (NET) arising from neuroendocrine cells which are found throughout the body in organs such as the pituitary, thyroid, adrenals, pancreas, the lungs and the gastrointestinal tract. In some cases, the ACTH-secreting tumor is an adenoma or adenocarcinoma. An adenoma is a benign tumor arising from epithelial tissue of glandular origin, having glandular characteristics, or both. Adenomas can arise in a variety of glandular tissues, such as adrenal glands and pituitary glands. Some adenomas arise from non-glandular tissues, but express glandular tissue structure. Although adenomas are benign, over time they may transform to become malignant, at which point they are called adenocarcinomas. However, even while benign, adenomas have the potential to cause serious health complications by compressing other structures (*e*.*g*., compressing brain or ocular nerve structures) or by producing large amounts of hormones (*e*.*g*., ACTH) in an unregulated, non-feedback-dependent manner. Even very small adenomas have the potential to secrete sufficient amounts of one or more hormones to cause clinical symptoms.

In embodiments that are disclosed but not claimed, the ACTH-secreting tumor can be a pituitary adenoma or pituitary adenocarcinoma. ACTH-secreting pituitary tumors can cause Cushing's Disease. As such, methods and compositions described herein can treat or relieve one or more symptoms of Cushing's Disease.

In some disclosed but non-claimed cases, the ACTH-secreting pituitary adenoma or adenocarcinoma has aberrant somatostatin receptor expression as compared to normal pituitary cells. Normal adult pituitary cells express somatostatin receptors SSTR1, 2, 3, and 5. In normal cells, SSTR5 is the most highly expressed somatostatin receptor sub-type, and SSTR4 is expressed at very low levels. Approximately 85% of ACTH-secreting pituitary adenomas express SSTR2 and SSTR5, while approximately 63% express SSTR1. *See,* Cuevas-Ramos & Fleseriu J. Mol. Endocrinol. (2014) 52, R223-R240.

In ACTH-secreting pituitary adenomas or adenocarcinomas, SSTR5 can remain the most abundant somatostatin receptor; however, somatostatin receptor expression (*e*.*g*., expression of any one of SSTR1-5, or a combination thereof) can be reduced. For example, expression of any one of SSTR1-5, or a combination thereof can be reduced due to hypercortisolism-induced down-regulation. In some cases, SSTR2 expression is reduced due to hypercortisolism induced downregulation. In some cases, the ACTH-secreting pituitary tumors increase somatostatin receptor expression (*e*.*g*., expression of any one of SSTR1-5, or a combination thereof) in response to GRA administration by blocking or mitigating hypercortisolism-induced down-regulation of SSTR expression. In some cases, SSTR2 expression is increased by GRA administration by blocking or mitigating hypercortisolism-induced down-regulation of SSTR2 expression.

The ACTH-secreting tumor is a neuroendocrine tumor and, in non-claimed embodiments, the ACTH-secreting tumor is a carcinoid tumor. Neuroendocrine tumors arise from cells of the endocrine or nervous systems. Neuroendocrine tumors can occur in any area or region of the body. However, neuroendocrine tumors are most often found in the intestine, pancreas, or lung. The ACTH-secreting tumor of the invention is a pancreatic neuroendocrine tumor. Neuroendocrine tumors can be classified as well-differentiated benign, well-differentiated uncertain, well-differentiated low-grade malignant, or poorly differentiated malignant tumors. The ACTH-secreting neuroendocrine tumor or carcinoid tumor can cause ectopic Cushing's Syndrome. As such, methods and compositions described herein can treat or relieve one or more symptoms of ectopic Cushing's Syndrome.

Neuroendocrine tumors that are disclosed but not claimed can include neuroendocrine tumors of the anterior pituitary; neuroendocrine thyroid tumors, such as medullary carcinomas; parathyroid tumors; thymus and mediastinal carcinoid tumors; pulmonary neuroendocrine tumors (*e*.*g*., bronchial tumors, pulmonary carcinoid tumors such as typical carcinoid, or atypical carcinoid tumors, small-cell lung cancer, and large cell neuroendocrine carcinomas of the lung); extrapulmonary small cell carcinomas; gastroenteropancreatic neuroendocrine tumors (*e*.*g*., foregut, midgut, or hindgut gastroenteropancreatic neuroendocrine tumors); neuroendocrine tumors of the liver or gallbladder; adrenal tumors; addrenomedullary tumors; pheochromocytomas; peripheral nervous system tumors (*e*.*g*., schwannoma, paraganglioma; or neuroblastoma); tumors of the breast; genitourinary tract tumors (*e*.*g*., urinary tract carcinoid tumors, ovarian tumors, neuroendocrine tumors of the cervix, or testicular tumors); Merkel cell carcinoma of the skin; multiple endocrine neoplasia type 1 or 2 tumors; tumors resulting from von Hippel-Lindau disease; neurofibromatosis type 1 tumors; tumors associated with tuberous sclerosis; tumors associated with or caused by Carney complex; or combinations thereof.

In some cases, the neuroendocrine or carcinoid tumor, such as one of the neuroendocrine or carcinoid tumors described herein, expresses one or more somatostatin receptor subtypes (*e*.*g*., one of SSTR1-5, or a combination thereof). In some cases, the neuroendocrine or carcinoid tumor exhibits downregulated expression of one or more somatostatin receptor subtypes (*e*.*g*., one of SSTR1-5, or a combination thereof). In some cases, the neuroendocrine or carcinoid tumor exhibits downregulated expression of one or more somatostatin receptor subtypes (*e*.*g*., one of SSTR1-5, or a combination thereof) in response to hypercortisolism. In some cases, the neuroendocrine or carcinoid tumor exhibits downregulated expression of SSTR2 in response to hypercortisolism. In some cases, administration of a GRA can increase expression of one or more somatostatin receptor subtypes (*e*.*g*., one of SSTR1-5, or a combination thereof) by blocking or mitigating hypercortisolism-induced down-regulation of SSTR expression. In some cases, SSTR2 expression is increased by GRA administration by blocking or mitigating hypercortisolism-induced down-regulation of SSTR2 expression.

### 2) Detection or diagnosis of ACTH-secreting tumors

ACTH-secreting tumors can be detected by a variety of means. For example, ACTH-secreting tumors generally result in the presence of excess ACTH, resulting in hypercortisolism. Thus, ACTH-secreting tumors can be identified based on the presence of symptoms of hypercortisolism (*e*.*g*., symptoms of Cushing's Disease or ectopic Cushing's Syndrome). Such symptoms include, but are not limited to one or more of the following: weight gain, high blood pressure, poor short term memory, poor concentration, irritability, excess hair growth, impaired immunological function, ruddy complexion, extra fat in the neck region, moon face, fatigue, red stretch marks, irregular menstruation, or a combination thereof. Symptoms of hypercortisolism can additionally or alternatively include without limitation one or more of the following: insomnia, recurrent infection, thin skin, easy bruising, weak bones, acne, balding, depression, hip or shoulder weakness, swelling of the extremities, diabetes mellitus, elevated white blood cell count, hypokalemic metabolic alkalosis, or a combination thereof.

In some cases, symptoms of hypercortisolism (*e*.*g*., Cushing's Disease or Cushing's Syndrome) are difficult to differentiate from other causes. Therefore, biochemical tests can be useful to determine the presence or absence of hypercortisolism, indicating the presence or absence of ACTH-secreting tumors respectively. Alternatively, biochemical tests can be used to directly detect the presence of excess ACTH. As such biochemical tests useful for the detection of ACTH-secreting tumors include, but are not limited to, tests that measure ACTH, cortisol, or a combination thereof. In some cases, salivary or blood serum cortisol levels are measured. In some cases, urinary free cortisol (LTFC), *e*.*g*., 24-hour LTFC is measured.

In some cases, ACTH is measured by bilateral inferior petrosal sinus sampling (BIPSS). In some cases, ACTH is measured by bilateral internal jugular vein sampling (BIJVS). In some cases, ACTH levels from BIPSS and/or BIJVS are compared to a peripherally obtained sample. The inferior petrosal sinus is where the pituitary gland drains. Therefore, a sample from this area that has high ACTH levels can suggest the presence of an ACTH-secreting tumor in the pituitary gland (*i*.*e*., Cushing's Disease). A low level of ACTH measured from the inferior pertrosal sinus can indicate the presence of an ACTH-secreting tumor that does not reside in the pituitary (*e*.*g*., ectopic Cushing's Syndrome). In some cases, detection of a central to periphery ACTH gradient can indicate whether the ACTH-secreting tumor is pituitary or non-pituitary.

In some cases, BIPSS and/or BIJVS is performed after administration of corticotropin-releasing hormone (CRH), desmopressin (DDAVP), or a combination thereof. These agents can increase ACTH secretion in active ACTH-producing pituitary tumors. In some cases, sampling is performed before and after administration of CRH, DDAVP, or a combination thereof. In some cases, a central to periphery ACTH gradient of more than 2 before and more than 3 after the administration of CRH or DDAVP indicates the presence of an ACTH secreting pituitary tumor. In some cases, gradients less than 2 before or less than 3 after the administration of CRH or DDAVP indicate a non-pituitary ACTH-secreting tumor.

ACTH-secreting tumors of pituitary or non-pituitary origin or location can be differentiated from other diseases or conditions that cause hypercortisolism or Cushing's-like symptoms by their ACTH-dependence. ACTH-dependent Cushing's Syndrome can indicate ectopic or pituitary Cushing's. ACTH-independent Cushing's Syndrome can indicate adrenal dysfunction (*e*.*g*., the presence of an adrenal adenoma or carcinoma). ACTH-independent Cushing's can be indicated by a low or undetectable plasma ACTH level in combination with a simultaneously elevated serum cortisol level. For example, a plasma ACTH level of less than 5 pg/mL (e.g., measured by an immunoradiometric assay) in a subject with high cortisol levels is suggestive of a primary adrenal tumor. In contrast, an ACTH level greater than 10-20 pg/mL is consistent with ACTH-dependent Cushing syndrome.

An overnight dexamethasone suppression test and/or high-dose dexamethasone test may be useful to diagnose the source of hypercortisolism, e.g., when baseline ACTH levels are indeterminate. These tests also help in determining whether a patient who has ACTH-dependent disease has pituitary-dependent or ectopic ACTH disease. For example, in the 8 mg overnight dexamethasone suppression test, individuals can ingest 8 mg dexamethasone orally in the evening of the first day, with measurement of cortisol levels early the next day. A baseline morning cortisol measurement can also obtained the morning prior to ingesting dexamethasone. Suppression of serum cortisol levels to less than 50% of baseline is suggestive of a pituitary source of ACTH rather than ectopic ACTH or primary adrenal disease.

As another example, in a 48-hour high-dose dexamethasone suppression test, patients ingest 2 mg dexamethasone every 6 hours for 8 doses. A decrease in urinary free cortisol of greater than 50% is suggestive of an anterior pituitary adenoma rather than ectopic ACTH or a primary adrenal tumor. The more stringent criterion of a 90% decrease in urinary free cortisol levels excludes the diagnosis of ectopic ACTH and has almost 100% specificity for anterior pituitary disease.

Testing with corticotropin releasing hormone (CRH) can be used in the differential diagnosis of ACTH-dependent Cushing syndrome. In most subjects with pituitary ACTH secretion, the intravenous administration of CRH causes a rise in plasma ACTH and cortisol levels. In subjects with ectopic secretion of ACTH, CRH generally does not affect ACTH or cortisol levels. In a CRH-test, ACTH and cortisol samples are obtained before administration of ovine CRH (oCRH), and subsequently at 15, 30, 45, 60, 90, and 120 minutes after administration of 1 mcg/kg of CRH. A rise of more than 20% in peak plasma cortisol level or a rise of more than 50% in peak ACTH level after oCRH is consistent with pituitary ACTH-dependent Cushing syndrome. Sensitivity and specificity are 91% and 95%, respectively, for cortisol measurements and 86% and 95% for ACTH measurements, respectively.

ACTH-secreting tumors can also be detected via imaging studies, such as computed tomography (CT) scanning, magnetic resonance imaging, scintigraphy, single photon emission computerized tomography (SPECT), ultrasound imaging, or a combination thereof. Generally, imaging studies are performed after biochemical tests have been performed and specific types of ACTH-secreting tumors are suspected or indicated. For example, if biochemical testing suggests ectopic ACTH-secreting tumors, then imaging can be used to scan for these tumors, e.g., in the chest or abdominal area where ectopic ACTH-secreting tumors most often arise. Similarly, if biochemical testing suggests pituitary ACTH secreting tumors, then imaging can be restricted to the pituitary gland and surrounding tissues.

For example, if a pituitary source of excess ACTH is suspected, subjects can undergo a contrast-enhanced magnetic resonance imaging (MRI) study of the pituitary. Unfortunately, normal-appearing pituitaries may occur in some patients with Cushing disease due to either diffuse hyperplasia of ACTH-producing cells or small microadenomas that do not appear on imaging studies. In the latter case, ACTH lateralization during a bilateral inferior petrosal sinus sampling (BIPSS) or bilateral internal jugular vein sampling (BIJVS) study may be useful in lateralizing an occult lesion and in guiding surgical therapy.

As another disclosed but non-claimed example, detectably labeled somatostatin or somatostatin analogues (SSAs) can be used to identify ACTH-secreting tumors (*e*.*g*., ectopic ACTH-secreting tumors such as neuroendocrine tumors). Such somatostatin or SSAs can include labeled octreotide. For example, ¹²³I-Tyr³-, ¹¹¹In-DTPA-*D*-Phe¹-octreotide, or [¹¹¹In-DTPA⁰]octreotide can be administered to a patient, allowed to bind to somatostatin receptor expressing ACTH-secreting tumors, and detected. Further examples of somatostatins and somatostatin analogues, including radionuclide labeled somatostatin analogues useful for imaging and/or radionuclide therapy include those disclosed in Baldelli *et al.* Front Endocrinol (Lausanne). 2014 Feb 7;5:7. In some cases, identification of ACTH-secreting tumors with a detectably labeled somatostatin or somatostatin analogue is augmented by administration of a glucocorticoid receptor antagonist (GRA) (*e*.*g*., mifepristone). In some cases, administration of a GRA can de-repress expression of one or more somatostatin receptors by the ACTH-secreting tumor cells (de Bruin et al, J Clin Endocrinol Metab, February 2012, 97(2):455-46).

### 3) Determining treatment efficacy

Any one or more of the foregoing detection or diagnostic methods described herein, or known generally in the art, can be used to assess treatment effect. In some disclosed but non-claimed embodiments, an ACTH-secreting tumor is treated in a subject in need thereof by administering a GRA and a somatostatin or somatostatin analog (SSA) in amounts effective to treat the ACTH-secreting tumor, and the treatment is monitored to determine efficacy. For example, efficacy can be indicated by detecting a decrease in ACTH levels, a decrease in hypercortisolism (*e*.*g*., a decrease in serum, urinary free, or salivary cortisol levels, or a decrease in symptoms of high cortisol levels), or a reduction in tumor burden. In some cases, the reduction in tumor burden is indicated by a reduction in size, mass, or volume of a tumor, or a reduction in symptoms caused by the tumor mass. For example, where a tumor mass physically impinges upon the optic nerve, effective treatment can be indicated by a reduction in visual field defects. In some cases, the reduction in tumor burden is indicated by a reduction in ACTH-secreting tumor cell proliferation or viability, or by an increase in ACTH-secreting tumor cell death.

### B. Glucocorticoid Receptor Antagonists

The methods of the present invention generally provide administering the specific glucocorticoid receptor antagonist mifepristone. As used herein, a specific glucocorticoid receptor antagonist refers to a composition or compound which inhibits any biological response associated with the binding of a glucocorticoid receptor to an agonist by preferentially binding to the glucocorticoid receptor rather than another nuclear receptor (NR). In some embodiments, the specific glucocorticoid receptor antagonist binds preferentially to glucocorticoid receptor rather than the mineralocorticoid receptor (MR), androgen receptor (AR), or progesterone receptor (PR). In an exemplary embodiment, the specific glucocorticoid receptor antagonist binds preferentially to glucocorticoid receptor rather than the mineralocorticoid receptor (MR). In another exemplary embodiment, the specific glucocorticoid receptor antagonist binds preferentially to glucocorticoid receptor rather than the progesterone receptor (PR). In another exemplary embodiment, the specific glucocorticoid receptor antagonist binds preferentially to glucocorticoid receptor rather than the androgen receptor (AR). In yet another exemplary embodiment, the specific glucocorticoid receptor antagonist binds preferentially to glucocorticoid receptor in comparison to MR and PR, MR and AR, or MR, PR, and AR.

In a related embodiment, the specific glucocorticoid receptor antagonist binds to the glucocorticoid receptor with a dissociation constant (K_{d}) that is or is less than 1/10^{th} the K_{d} (*i.e.,* at least 10x greater affinity than) for another nuclear receptor *(e.g.,* AR; MR; PR; MR and PR; MR and AR; or MR; PR; and AR). In another embodiment, the specific glucocorticoid receptor antagonist binds to the glucocorticoid receptor with a dissociation constant (K_{d}) that is or is less than 1/100^{th} the K_{d} (*i.e.,* at least 100x greater affinity than) for the other nuclear receptor (e.g., AR; MR; PR; MR and PR; MR and AR; or MR; PR; and AR). In another embodiment, the specific glucocorticoid receptor antagonist binds to the glucocorticoid receptor with a dissociation constant (K_{d}) that is or is less than 1/1000^{th} the K_{d} (*i.e.,* at least 1000x greater affinity than) for the other nuclear receptor *(e.g.,* AR; MR; PR; MR and PR; MR and AR; or MR; PR; and AR).

### 1) Exemplary Glucocorticoid Receptor Antagonists

Generally, treatment is provided by administering an effective amount of the glucocorticoid receptor antagonist (GRA) mifepristone and the SSA octreotide. Disclosed herein but not claimed are classes of exemplary GRAs and specific members of such classes.

### a) GRAs Having a Steroidal Backbone

In some embodiments, an effective amount of a GRA with a steroidal backbone, namely mifepristone, is administered to a subject for treatment of Cushing's syndrome in a patient having an ACTH-secreting pancreatic neuroendocrine tumor. Steroidal GRAs can be obtained by modification of the basic structure of glucocorticoid agonists, i.e., varied forms of the steroid backbone. The structure of cortisol can be modified in a variety of ways. The two most commonly known classes of structural modifications of the cortisol steroid backbone to create GRAs include modifications of the 11-β hydroxy group and modification of the 17-β side chain (*See, e.g.,* Lefebvre, J. Steroid Biochem. 33:557-563, 1989).

Non-claimed examples of steroidal GR antagonists include androgen-type steroidal compounds as described in U.S. Pat. No. 5,929,058, and the compounds disclosed in U.S. Pat. Nos. 4,296,206; 4,386,085; 4,447,424; 4,477,445; 4,519,946; 4,540,686; 4,547,493; 4,634,695; 4,634,696; 4,753,932; 4,774,236; 4,808,710; 4,814,327; 4,829,060; 4,861,763; 4,912,097; 4,921,638; 4,943,566; 4,954,490; 4,978,657; 5,006,518; 5,043,332; 5,064,822; 5,073,548; 5,089,488; 5,089,635; 5,093,507; 5,095,010; 5,095,129; 5,132,299; 5,166,146; 5,166,199; 5,173,405; 5,276,023; 5,380,839; 5,348,729; 5,426,102; 5,439,913; 5,616,458, 5,696,127, and 6,303,591. Such non-claimed steroidal GR antagonists include cortexolone, dexamethasone-oxetanone, 19-nordeoxycorticosterone, 19-norprogesterone, cortisol-21-mesylate; dexamethasone-21-mesylate, 11β-(4-dimethylaminoethoxyphenyl)-17α-propynyl-17β-hydroxy-4,9-estradien-3-one (RU009), and (17α)-17-hydroxy-19-(4-methylphenyl)androsta-4,9(11)-dien-3-one (RU044).

Other non-claimed examples of steroidal antiglucocorticoids are disclosed in Van Kampen et al. (2002) Eur. J. Pharmacol. 457(2-3):207, WO 03/043640, EP 0 683 172 B1, and EP 0 763 541 B1. EP 0 763 541 B1 and Hoyberg et al., Int'l J. of Neuro-psychopharmacology, 5:Supp. 1, S148 (2002); disclose the non-claimed compound (11β,17β)-11-(1,3-benzodioxol-5-yl)-17-hydroxy-17-(1-propynyl)estra-4,9-dien-3-one (ORG 34517) which may be administered in an amount effective to treat an ACTH-secreting tumor in a subject.

### i. Removal or Substitution of the 11- β Hydroxy Group

Glucocorticoid antagonists with modified steroidal backbones comprising removal or substitution of the 11-β hydroxy group are administered in one embodiment of the invention. This class includes natural GRAs, including cortexolone, progesterone and testosterone derivatives, and synthetic compositions, such as mifepristone (Lefebvre, *et al. supra*)*.* 11-β aryl steroid backbone derivatives, such as mifepristone, can be devoid of progesterone receptor (PR) binding activity (Agarwal, FEBS 217:221-226, 1987). In the invention, the 11-β phenyl-aminodimethyl steroid backbone derivative mifepristone, which is both an effective anti-glucocorticoid and anti-progesterone agent, is administered. These compositions can act as reversibly-binding steroid receptor antagonists. For example, when bound to a 11-β phenyl-aminodimethyl steroid, the steroid receptor can be maintained in a conformation that cannot bind its natural ligand, such as cortisol in the case of GR (Cadepond, 1997, *supra*)*.*

Synthetic 11-beta phenyl-aminodimethyl steroids include mifepristone, also known as RU486, or 17-β-hydrox-11-β-(4-dimethyl-aminophenyl)17-α-(1-propynyl)estra-4,9-dien-3-one). Mifepristone has been shown to be a powerful antagonist of both the progesterone and glucocorticoid (GR) receptors. Thus, in all embodiments of the invention, the GRA administered to treat an ACTH-secreting tumor is mifepristone, or a salt, tautomer, or derivative thereof.

Another disclosed but non-claimed 11-β phenyl-aminodimethyl steroid shown to have GR antagonist effects includes the dimethyl aminoethoxyphenyl derivative RU009 (RU39.009), 11-β-(4-dimethyl-aminoethoxyphenyl)-17-α-(propynyl-17-β-hydroxy-4,9-estradien-3-one) (see Bocquel, J. Steroid Biochem. Molec. Biol. 45:205-215, 1993). Another disclosed but non-claimed GR antagonist related to RU486 is RU044 (RU43.044) 17-β-hydrox-17-α-19-(4-methyl-phenyl)-androsta-4,9(11)-dien-3-one) (Bocquel, 1993, *supra*)*. See* also Teutsch, Steroids 38:651-665, 1981; U.S. Pat. Nos. 4,386,085 and 4,912,097.

### ii. Modification of the 17-beta Side Chain Group

Steroidal antiglucocorticoids which can be obtained by various structural modifications of the 17-β side chain are disclosed herein but are not claimed. This class includes synthetic antiglucocorticoids such as dexamethasone-oxetanone, various 17, 21-acetonide derivatives and 17-beta-carboxamide derivatives of dexamethasone (Lefebvre, 1989, *supra;* Rousseau, Nature 279:158-160, 1979).

### iii. Other Steroid Backbone Modifications

GRAs disclosed herein but not claimed further include any steroid backbone modification which effects a biological response resulting from a GR-agonist interaction. Steroid backbone antagonists can be any natural or synthetic variation of cortisol, such as adrenal steroids missing the C-19 methyl group, such as 19-nordeoxycorticosterone and 19-norprogesterone (Wynne, Endocrinology 107:1278-1280, 1980).

In general, the 11- βside chain substituent, and particularly the size of that substituent, can play a key role in determining the extent of a steroid's antiglucocorticoid activity. Substitutions in the A ring of the steroid backbone can also be important. For example, 17-hydroxypropenyl side chains can, in some cases, decrease antiglucocorticoid activity in comparison to 17-propynyl side chain containing compounds.

Additional glucocorticoid receptor antagonists known in the art but not claimed include 21-hydroxy-6,19-oxidoprogesterone (*See* Vicent, Mol. Pharm. 52:749-753, 1997), Org31710 (*See* Mizutani, J Steroid Biochem Mol Biol 42(7):695-704, 1992), RU43044, RU40555 (*See* Kim, J Steroid Biochem Mol Biol. 67(3):213-22, 1998), and RU28362.

### b) Non-Steroidal Anti-Glucocorticoids as Antagonists

Non-steroidal glucocorticoid antagonists (GRAs) to treat ACTH-secreting tumors in a subject are disclosed but are not claimed. These include synthetic mimetics and analogs of proteins, including partially peptidic, pseudopeptidic and non-peptidic molecular entities. For example, oligomeric peptidomimetics include (α-β-unsaturated) peptidosulfonamides, N-substituted glycine derivatives, oligo carbamates, oligo urea peptidomimetics, hydrazinopeptides, oligosulfones and the like (*See, e.g.,* Amour, Int. J. Pept. Protein Res. 43:297-304, 1994; de Bont, Bioorganic &Medicinal Chem. 4:667-672, 1996).

Examples of non-steroidal GR antagonists include the GR antagonist compounds disclosed in U.S. Pat. Nos. 5,696,127; 6,570,020; and 6,051,573; the GR antagonist compounds disclosed in US Patent Application 20020077356, the glucocorticoid receptor antagonists disclosed in Bradley et al., J. Med. Chem. 45, 2417-2424 (2002), *e.g.,* 4α(S)-benzyl-2(R)-chloroethynyl-1,2,3,4,4α,9,10,10α(R)-octahydro-phenanthrene-2,7-diol ("CP 394531") and 4α(S)-benzyl-2(R)-prop-1-ynyl-1,2,3,4,4α,9,10,10α(R)-octahydro-phenanthrene-2,7-diol ("CP 409069"); and the compounds disclosed in PCT International Application No. WO 96/19458, which describes non-steroidal compounds which are high-affinity, highly selective antagonists for steroid receptors, such as 6-substituted-1,2-dihydro-N-protected-quinolines.

### C. Somatostatin Receptor Ligands

ACTH-secreting tumors are treated with an effective amount of the somatostatin analog (SSA) octreotide. Thus, the ACTH-secreting tumor is treated with effective amounts of the GRA mifepristone and the somatostatin analog (SSA) octreotide.

Exemplary non-claimed somatostatin receptor ligands include, without limitation, peptide somatostatin receptor ligands, such as those described in U.S. Patent No. 8,946,154. Exemplary non-claimed somatostatin receptor ligands further include, without limitation, somatostatin polypeptides from *Oncorhynchus mykiss* and analogs or derivatives thereof, such as those described in U.S. Patent No. 6,818,739. Exemplary non-claimed somatostatin receptor ligands further include, without limitation, antibodies that bind to, or bind to and activate one or more somatostatin receptor subtypes (e.g., any one of SSTR1-5, or a combination thereof). Exemplary non-claimed somatostatin receptor ligands further include, without limitation, non-peptide somatostatin receptor ligands such as those described in U.S. Patent No. 7,189856. Exemplary non-claimed somatostatin receptor ligands further include, without limitation, the somatostatin receptor ligands described in U.S. Patent No. 6,358,941.

Exemplary somatostatin receptor ligands further include, without limitation, selective somatostatin receptor ligands. For example, the somatostatin receptor ligand can be selective for (e.g., selectively binds to, or selectively activates) one of SSTR1-5. In some cases, the somatostatin receptor ligand is selective for (e.g., selectively binds to, or selectively activates) SSTR1. In some cases, the somatostatin receptor ligand is selective for SSTR2. Exemplary In some cases, the somatostatin receptor ligand is selective for (e.g., selectively binds to, or selectively activates) SSTR3. In some cases, the somatostatin receptor ligand is selective for (e.g., selectively binds to, or selectively activates) SSTR4. In some cases, the somatostatin receptor ligand is selective for (e.g., selectively binds to, or selectively activates) SSTR5.

In some cases, the somatostatin receptor ligand is selective for (e.g., selectively binds to, or selectively activates) two somatostatin receptors selected from the group consisting of SSTR1-5. For example, the somatostatin receptor ligand can be selective for SSTR1 and 4. As another example, the somatostatin receptor ligand can be selective for SSTR2 and 5. In some cases, the somatostatin receptor ligand is selective for (e.g., selectively binds to, or selectively activates) three somatostatin receptors selected from the group consisting of SSTR1-5. In some cases, the somatostatin receptor ligand is selective for (e.g., selectively binds to, or selectively activates) four somatostatin receptors selected from the group consisting of SSTR1-5. Exemplary selective non-claimed somatostatin receptor ligands include, without limitation, those described in Rohrer et al., 1998, Science 282:737. Exemplary selective non-claimed somatostatin receptor ligands further include, without limitation, those described in U.S. Patent Appl. Pub. No. 2006/008299.

In all cases, the somatostatin receptor ligand is octreotide. In some cases, the somatostatin receptor ligand is coupled to a detectable label or a cytotoxic agent. Exemplary detectable labels include spin labels, fluorescent labels, and radionuclides. Exemplary cytotoxic agents include radionuclides and cytotoxic chemotherapeutics. Exemplary somatostatin receptor ligands coupled to a radionuclide include, but are not limited to ¹²³I-Tyr³-octreotide, ¹¹¹In-DTPA-*D*-Phe¹-octreotide, [¹¹¹In-DTPA⁰]octreotide, or [⁹⁰Y-DOTA, Tyr³]octreotide.

### D. Methods of Administration

An adrenocorticotropic hormone (ACTH)-secreting tumor is treated in a subject in need thereof, by sequentially administering to the subject i) the glucocorticoid receptor antagonist (GRA) mifepristone; and ii) the somatostatin analog (SSA) octreotide, in amounts effective to reduce secretion of ACTH by the tumor, wherein the SSA octreotide is administered first, followed by a second administration of the GRA mifepristone.

In some cases, the first administration is followed immediately, or nearly immediately, by the second administration. Alternatively, the second administration may be delayed by seconds, minutes, hours, days, or weeks. In some cases, the SSA octreotide is repeatedly administered for a period of time (e.g., hours, days, or weeks), and then the GRA mifepristone is administered (e.g., alone or in combination with the SSA). In some cases, after simultaneous or sequential GRA and SSA administration is performed for a period of time (e.g., hours, days, weeks, or months), the GRA administration is continued for a period of time (e.g., hours, days, weeks, or months) and SSA administration is discontinued for a period of time (e.g., hours, days, weeks, or months).

GRAs can be administered orally. For example, the GRA mifepristone can be administered as a pill or liquid formulation as described herein. Alternatively, GRAs can be provided via parenteral administration. For example, the GRA mifepristone can be administered intravenously (e.g., by injection or infusion). Similarly, the SSA octreotide can be administered orally, e.g., as a pill or liquid formulation. Alternatively, the SSA octreotide can be administered via parenteral administration, e.g., intravenously, subcutaneously, or intramuscularly. Additional methods of administration of the compounds described herein, and pharmaceutical compositions or formulations thereof, are described below in section IV.B.

### IV. Disclosed but Non-Claimed Pharmaceutical Compositions

The present disclosure provides a pharmaceutical composition including a compound disclosed herein and a pharmaceutically acceptable excipient. In some embodiments, the present disclosure provides a pharmaceutical composition including a glucocorticoid receptor antagonist disclosed herein and a pharmaceutically acceptable excipient. In some embodiments, the present disclosure provides a pharmaceutical composition including a somatostatin receptor ligand disclosed herein and a pharmaceutically acceptable excipient. In some embodiments, the present invention provides a pharmaceutical composition including a glucocorticoid receptor antagonist and a somatostatin receptor ligand as disclosed herein and a pharmaceutically acceptable excipient.

### A. Formulation

The compositions can be prepared in a wide variety of oral, parenteral and topical dosage forms. Oral preparations include tablets, pills, powder, dragees, capsules, liquids, lozenges, cachets, gels, syrups, slurries, suspensions, etc., suitable for ingestion by the patient. The compositions can also be administered by injection, that is, intravenously, intramuscularly, intracutaneously, subcutaneously, intraduodenally, or intraperitoneally. Also, the compositions described herein can be administered by inhalation, for example, intranasally. Additionally, the compositions can be administered transdermally. The compositions can also be administered by intraocular, intravaginal, and intrarectal routes including suppositories, insufflation, powders and aerosol formulations (for examples of steroid inhalants, see Rohatagi, J. Clin. Pharmacol. 35:1187-1193, 1995; Tjwa, Ann. Allergy Asthma Immunol. 75:107-111, 1995). Accordingly, the present disclosure provides pharmaceutical compositions including a pharmaceutically acceptable carrier or excipient and a compound disclosed herein.

For preparing pharmaceutical compositions from the compounds disclosed herein, pharmaceutically acceptable carriers can be either solid or liquid. Solid form preparations include powders, tablets, pills, capsules, cachets, suppositories, and dispersible granules. A solid carrier can be one or more substances, which may also act as diluents, flavoring agents, binders, preservatives, tablet disintegrating agents, or an encapsulating material. Details on techniques for formulation and administration are well described in the scientific and patent literature, see, e.g., the latest edition of Remington's Pharmaceutical Sciences, Maack Publishing Co, Easton PA ("Remington's").

In powders, the carrier is a finely divided solid, which is in a mixture with the finely divided active component. In tablets, the active component is mixed with the carrier having the necessary binding properties in suitable proportions and compacted in the shape and size desired. The powders and tablets preferably contain from 5% or 10% to 70% of the compounds disclosed herein.

Suitable solid excipients include, but are not limited to, magnesium carbonate; magnesium stearate; talc; pectin; dextrin; starch; tragacanth; a low melting wax; cocoa butter; carbohydrates; sugars including, but not limited to, lactose, sucrose, mannitol, or sorbitol, starch from corn, wheat, rice, potato, or other plants; cellulose such as methyl cellulose, hydroxypropylmethyl-cellulose, or sodium carboxymethylcellulose; and gums including arabic and tragacanth; as well as proteins including, but not limited to, gelatin and collagen. If desired, disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound (i.e., dosage). Pharmaceutical preparations described herein can also be used orally using, for example, push-fit capsules made of gelatin, as well as soft, sealed capsules made of gelatin and a coating such as glycerol or sorbitol. Push-fit capsules can contain the compounds of the present invention mixed with a filler or binders such as lactose or starches, lubricants such as talc or magnesium stearate, and, optionally, stabilizers. In soft capsules, the compounds disclosed herein may be dissolved or suspended in suitable liquids, such as fatty oils, liquid paraffin, or liquid polyethylene glycol with or without stabilizers.

For preparing suppositories, a low melting wax, such as a mixture of fatty acid glycerides or cocoa butter, is first melted and the compounds disclosed herein are dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution, e.g., in aqueous polyethylene glycol solution.

Aqueous solutions suitable for oral use can be prepared by dissolving one or more compounds disclosed herein in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

Oil suspensions can be formulated by suspending the compounds disclosed herein in a vegetable oil, such as arachis oil, olive oil, sesame oil or coconut oil, or in a mineral oil such as liquid paraffin; or a mixture of these. The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto, J. Pharmacol. Exp. Ther. 281:93-102, 1997. The pharmaceutical formulations can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent.

One or more compositions can also be delivered as microspheres for slow release in the body. One or more compositions can be delivered as a gel depot for slow release in the body. For example, microspheres can be formulated for administration via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res. 12:857-863, 1995); or, as microspheres for oral administration (see, e.g., Eyles, J. Pharm. Pharmacol. 49:669-674, 1997). Both transdermal and intradermal routes afford constant delivery for weeks or months. The somatostatin analogue can be in the form of a microsphere powder for suspension in a liquid diluent and injection. In some cases, the suspended microsphere formulation provides a long acting release administration form. The somatostatin analogue can be in the form of an autogel, *e.g.,* a supersaturated gel of active ingredient and water. In some cases, the autogel formulation provides a sustained release administration form.

The compositions can be formulated for parenteral administration, such as intravenous (IV) administration or administration into a body cavity or lumen of an organ. The formulations for administration will commonly comprise a solution of the compositions dissolved in a pharmaceutically acceptable carrier. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of inj ectables. These solutions are sterile and generally free of undesirable matter. These formulations may be sterilized by conventional, well known sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of the compositions in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated according to the known art using those suitable dispersing or wetting agents and suspending agents. The sterile injectable preparation can also be a sterile injectable solution or suspension in a nontoxic parenterally-acceptable diluent or solvent, such as a solution of 1,3-butanediol.

The formulations of the compositions can be delivered by the use of liposomes which fuse with the cellular membrane or are endocytosed, i.e., by employing ligands attached to the liposome, or attached directly to the oligonucleotide, that bind to surface membrane protein receptors of the cell resulting in endocytosis. By using liposomes, particularly where the liposome surface carries ligands specific for target cells, or are otherwise preferentially directed to a specific organ, one can focus the delivery of the compositions into the target cells in vivo. (See, e.g., Al-Muhammed, J. Microencapsul. 13:293-306, 1996; Chonn, Curr. Opin. Biotechnol. 6:698-708, 1995; Ostro, Am. J. Hosp. Pharm. 46:1576-1587, 1989).

Lipid-based drug delivery systems include lipid solutions, lipid emulsions, lipid dispersions, self-emulsifying drug delivery systems (SEDDS) and self-microemulsifying drug delivery systems (SMEDDS). In particular, SEDDS and SMEDDS are isotropic mixtures of lipids, surfactants and co-surfactants that can disperse spontaneously in aqueous media and form fine emulsions (SEDDS) or microemulsions (SMEDDS). Lipids useful in the formulations described herein include any natural or synthetic lipids including, but not limited to, sesame seed oil, olive oil, castor oil, peanut oil, fatty acid esters, glycerol esters, Labrafil^{®}, Labrasol^{®}, Cremophor^{®}, Solutol^{®}, Tween^{®}, Capryol^{®}, Capmul^{®}, Captex^{®}, and Peceol^{®}.

### B. Administration

One or more compounds or compositions described herein can be delivered by any suitable means, including oral, parenteral and topical methods. Transdermal administration methods, by a topical route, can be formulated as applicator sticks, solutions, suspensions, emulsions, gels, creams, ointments, pastes, jellies, paints, powders, and aerosols.

The pharmaceutical preparation is preferably in unit dosage form. In such form the preparation is subdivided into unit doses containing appropriate quantities of the compounds and compositions. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The compounds and compositions can be co-administered with other agents. Co-administration includes administering the compound or composition within 0.5, 1, 2, 4, 6, 8, 10, 12, 16, 20, or 24 hours of the other agent. Co-administration also includes administering simultaneously, approximately simultaneously (e.g., within about 1, 5, 10, 15, 20, or 30 minutes of each other), or sequentially in any order. Moreover, the compounds and compositions can each be administered once a day, or two, three, or more times per day so as to provide the preferred dosage level per day.

In some embodiments, co-administration can be accomplished by co-formulation, i.e., preparing a single pharmaceutical composition including the compounds and compositions and any other agent. Alternatively, the various components can be formulated separately.

The compounds and compositions, and any other agents, can be present in any suitable amount, and can depend on various factors including, but not limited to, weight and age of the subject, state of the disease, etc. Suitable dosage ranges include from about 0.1 mg to about 10,000 mg, or about 1 mg to about 1000 mg, or about 10 mg to about 750 mg, or about 25 mg to about 500 mg, about 50 mg to about 250 mg, or about 75 mg to about 150 mg. Suitable dosages also include about 1, 5, 10, 20, 30, 40, 50, 60, 70, 80, 90, 100, 200, 300, 400, 500, 600, 700, 800, 900 or 1000 mg.

Glucorticoid receptor antagonists (GRAs) can be administered simultaneously or sequentially with a somatostatin receptor ligand *(e.g.,* an analog thereof) at a dose of from about 0.1 mg to about 10,000 mg, about 1 mg to about 1000 mg, about 10 mg to about 750 mg, about 25 mg to about 500 mg, about 50 mg to about 250 mg, or about 75 mg to about 150 mg of the GRA. In some cases, GRAs can be administered simultaneously or sequentially with a somatostatin receptor ligand *(e.g.,* an analog thereof) at a dose of about 1, 5, 10, 20, 30, 40, 50, 60, 70, 80, 90, 100, 200, 300, 400, 500, 600, 700, 800, 900 or 1000 mg of the GRA. In some cases, GRAs can be administered simultaneously or sequentially with a somatostatin receptor ligand *(e.g.,* an analog thereof) at a dose of about 0.1, 0.25, 0.5, 0.75, 1, 1.5, 2, 2.5, 3, 4, 5, 10, 15, 20, 25, 50, 75, 100, 125, or 150 mg/kg of the GRA. In some cases, one or more of the foregoing GRA dosages or a dose within one of the foregoing GRA dose ranges can be administered about four times per day, three times per day, once per day, semi-weekly, weekly, bi-weekly, or monthly. In some cases, a subject is administered a high dose *(e.g.,* 500 mg or more) of GRA for a period of time *(e.g.,* twice per day for one week) and then administered a low dose *(e.g.,* 100 mg or less) of GRA for a period of time.

Somatostatin receptor ligands *(e.g.,* an SSA) can be administered simultaneously or sequentially with a GRA at a dose of from about 0.1 mg to about 10,000 mg, about 1 mg to about 1000 mg, about 10 mg to about 750 mg, about 25 mg to about 500 mg, about 50 mg to about 250 mg, or about 75 mg to about 150 mg of the somatostatin receptor ligand. In some cases, the somatostatin receptor ligand can be administered simultaneously or sequentially with a GRA at a dose of about 1, 5, 10, 20, 30, 40, 50, 60, 70, 80, 90, 100, 200, 300, 400, 500, 600, 700, 800, 900 or 1000 mg of the somatostatin receptor ligand. In some cases, somatostatin receptor ligand can be administered simultaneously or sequentially with a GRA at a dose of about 0.1, 0.25, 0.5, 0.75, 1, 1.5, 2, 2.5, 3, 4, 5, 10, 15, 20, 25, 50, 75, 100, 125, or 150 mg/kg of the somatostatin receptor ligand. In some cases, one or more of the foregoing dosages of somatostatin receptor ligand or a dose within one of the foregoing dose ranges of somatostatin receptor ligand can be administered about four times per day, three times per day, once per day, semi-weekly, weekly, bi-weekly, or monthly. In some cases, a subject is administered a high dose (e.g., 500 mg or more) of somatostatin receptor ligand for a period of time (e.g., twice per day for one week) and then administered a low dose (*e.g.,* 100 mg or less) of somatostatin receptor ligand for a period of time.

The composition can also contain other compatible therapeutic agents. The compounds described herein can be used in combination with one another, with other active agents known to be useful in antagonizing a glucocorticoid receptor, or with adjunctive agents that may not be effective alone, but may contribute to the efficacy of the active agent.

The terms and expressions which have been employed herein are used as terms of description and not of limitation, and there is no intention in the use of such terms and expressions of excluding equivalents of the features shown and described, or portions thereof, it being recognized that various modifications are possible within the scope of the invention claimed. Moreover, any one or more features of any embodiment of the invention may be combined with any one or more other features of any other embodiment of the invention, without departing from the scope of the invention.

### V. Examples

### Example 1: Treatment of a subject with an ectopic ACTH-secreting tumor

A human patient with an ectopic ACTH-secreting pancreatic neuroendocrine tumor metastatic to liver gastrinoma presented with symptoms of ectopic Cushing's Syndrome. The patient was treated with the maximum recommended dose of octreotide long-acting release (LAR), a partial biochemical response was noted (ACTH decreased from 517 pg/mL (113.7 pmol/L) to 345 pg/mL (75.9 pmol/L)), but the Cushing's symptoms were not controlled. After three months of therapy with octreotide LAR, the patient was enrolled in a 24-week, phase 3 clinical trial of mifepristone (MIFE) for inoperable hypercortisolemia.

Prior to the start of MIFE, baseline urinary-free cortisol (UFC) was 2250 mcg/24 hours (6207 nmol/24 hours) and ACTH was 345 pg/mL (75.9 pmol/L). Late-night salivary cortisol (1.71 mcg/dL (47.2 nmol/L)) and serum cortisol (46 mcg/dL (1256 nmol/L)) were also elevated (Table 1). At the time of enrollment, the patient had overtly cushingoid features, including moon facies, plethora, and enlarged dorsocervical and supraclavicular fat pads; purple striae; bruising; edema; and proximal muscle weakness that was so severe that he was unable to rise from a chair without use of his hands. He also had ongoing diabetes, depression, and hypertension associated with hypokalemia.

Mifepristone was initiated at a daily dose of 300 mg and gradually increased to 1200 mg per protocol. The patient continued to receive octreotide LAR throughout the duration of the trial. By week 4, insulin therapy was discontinued and by week 12, his cushingoid features essentially resolved. In addition to clinical improvement, a dramatic decrease in cortisol and ACTH was noted during therapy with mifepristone and octreotide LAR (Figure 1, Table 1). At week 20, mifepristone was briefly stopped for significant fatigue, low appetite, and nausea. Mifepristone was then resumed at a daily dose of 900 mg and 1 week later reduced to 600 mg; no changes were made to octreotide LAR dose. At week 24, his UFC and ACTH levels were 434 mcg/24 hours (1198.7 nmol/24 hours) and 304 pg/mL (66.9 pmol/L), respectively, and mifepristone was stopped per study protocol. During withdrawal of mifepristone, the cortisol and ACTH rose, and 12 days after mifepristone was stopped, clinical signs and symptoms of EAS returned. After 2 weeks, his UFC and ACTH increased to 4716 mcg/24 hours (13016 nmol/24 hours) and 652 pg/mL (143.4 pmol/L), respectively (Figure 1, Table 1). Mifepristone was resumed for an additional 12-month extension period. Octreotide LAR was discontinued after 2 months and the patient continued with mifepristone for control of his CS-related symptoms. The collection of cortisol and ACTH data was less frequent during the extension study. At the time octreotide was discontinued, the patient's ACTH and serum cortisol were 652 pg/mL (143.4 pmol/L) and 67.8 mcg/dL (1871 nmol/L), respectively. After 12 months in the extension phase, substantial increases in ACTH (3738 pg/mL (822.4 pmol/L)), serum cortisol (135.2 mcg/dL (3732 nmol/L)), and UFC (10716.5 mcg/24 hours (29577.5 nmol/24 hours)) were observed.

**Table 1: ACTH, UFC, serum cortisol, and salivary cortisol levels during the course of treatment**

| Test (normal range) | Baseline (before MIFE) | Week 6 | Week 10 | Week 16 | Week 24 | 2-week follow-up (off MIFE) |
|---|---|---|---|---|---|---|
| ACTH, pg/mL (7-50 pg/mL) | 345 | 279 | 188 | 250 | 304 | 652 |
| UFC, mcg/24 h (2.0-42.4 mcg/24 h) | 2250 | 1536 | 104 | 122 | 434 | 4776 |
| Serum cortisol, mcg/dL (8 AM, 4.0-22.0 mcg/dL) | 46 | 41 | 31 | 31 | 37 | 68 |
| Late-night salivary cortisol, mcg/dL (10 PM-11 PM, ≤0.09 mcg/dL) | 1.71 | 2.18 | 0.56 | 0.73 | 1.49 | 4.91 |

## Claims

1. Mifepristone and octreotide for use in the treatment of Cushing's syndrome in a patient having an adrenocorticotropic hormone (ACTH)-secreting pancreatic neuroendocrine tumor, wherein the octreotide is administered first followed by a second administration of mifepristone.

2. The mifepristone and the octreotide for use according to claim 1, wherein the subject suffers from Cushing's Disease or an ectopic ACTH Syndrome.

3. The mifepristone and octreotide for use according to claim 1, wherein the octreotide is to be administered in a sustained release formulation.

4. The mifepristone and the octreotide for use according to claim 1, wherein the octreotide comprises a therapeutic radionuclide.

5. The mifepristone and the octreotide for use according to claim 4, wherein the therapeutic radionuclide is selected from the group consisting of ¹¹¹In, ⁹⁰Y, ¹⁷⁷Lu, and ²¹³Bi.

## Patentansprüche

1. Mifepriston und Octreotid zur Verwendung bei der Behandlung des Cushing-Syndroms bei einem Patienten mit einem ein adrenocorticotropes Hormon (ACTH) sezernierenden neuroendokrinen Tumor der Bauchspeicheldrüse, wobei zunächst das Octreotid verabreicht wird, gefolgt von einer zweiten Verabreichung von Mifepriston.

2. Mifepriston und Octreotid zur Verwendung nach Anspruch 1, wobei das Subjekt an der Cushing-Krankheit oder einem ektopischen ACTH-Syndrom leidet.

3. Mifepriston und Octreotid zur Verwendung nach Anspruch 1, wobei das Octreotid in einer Formulierung mit verzögerter Freisetzung zu verabreichen ist.

4. Mifepriston und Octreotid zur Verwendung nach Anspruch 1, wobei das Octreotid ein therapeutisches Radionuklid umfasst.

5. Mifepriston und Octreotid zur Verwendung nach Anspruch 4, wobei das therapeutische Radionuklid aus der Gruppe bestehend aus ¹¹¹In, ⁹⁰Y, ¹⁷⁷Lu und ²¹³Bi ausgewählt ist.

## Revendications

1. Mifépristone et octréotide destinés à être utilisés dans le traitement du syndrome de Cushing chez un patient comportant une tumeur neuroendocrine pancréatique sécrétant l'hormone adrénocorticotrope (ACTH), l'octréotide étant administré en premier, suivi d'une deuxième administration de mifépristone.

2. Mifépristone et octréotide destinés à être utilisés selon la revendication 1, le sujet souffrant de la maladie de Cushing ou d'un syndrome ACTH ectopique.

3. Mifépristone et octréotide destinés à être utilisés selon la revendication 1, l'octréotide devant être administré dans une formulation à libération prolongée.

4. Mifépristone et octréotide destinés à être utilisés selon la revendication 1, l'octréotide comprenant un radionucléide thérapeutique.

5. Mifépristone et octréotide destinés à être utilisés selon la revendication 4, le radionucléide thérapeutique étant sélectionné dans le groupe constitué de ¹¹¹In, ⁹⁰Y, ¹⁷⁷Lu et ²¹³Bi.
